# EUROPÄISCHE PATENTANMELDUNG

(11) **EP 3 006 993 A1**
(43) Veröffentlichungstag der Anmeldung: **13.04.2016**
(21) Anmeldenummer: 15002845.4
(22) Anmeldetag: 06.10.2015
(51) Int. Cl.: G02C 7/10, A61F 9/06, G02F 1/1345

(54) **ELEKTRO-OPTISCHES BLENDSCHUTZGLAS**

(30) Priorität: 06.10.2014 CH 15172014
(71) Anmelder: castelberg design GmbH, 8045 Zürich (CH)
(72) Erfinder: castelberg design GmbH, 8045 Zürich (CH)
(74) Vertreter: Alder, Hans Rudi

(57) **Zusammenfassung**

Ein elektro-optisches und für Schutz-, Sport- und Sonnenbrillen besonders geeignetes Blendschutzglas (21) weist eine LCD (17) mit mindestens einem aussenseitigen (29) und einem innenseitigen Polarisator (29') auf und umfasst eine elektronische Regelschaltung (11). Diese Regelschaltung (11) ist in der LCD (17) des Blendschutzglases (21) vollständig integriert, d.h. in einer Kavität (30) im Innern dieses Blendschutzglases (21) angeordnet. Dieser Anordnung erlaubt eine kostengünstige Herstellung von elektro-optischen Schutz-, Sport- und Sonnenbrillen mit autarken Gläsern, erhöht deren Designfreiheit, vereinfacht deren Montage und vermindert deren Störungsanfälligkeit.

## Beschreibung

Die vorliegende Erfindung betrifft ein elektro-optisches Blendschutzglas, insbesondere für Schutz-, Sport- und Sonnenbrillen, mit einer elektronischen Regelschaltung für eine LCD gemäss Oberbegriff des Anspruchs 1.

Elektrooptische Schutz-, Sport- und Sonnenbrillen (im Folgenden auch Blendschutzbrillen genannt) sind hinlänglich bekannt und weisen mindestens ein sich selbsttätig abdunkelndes Brillenglas (im Folgenden auch Blendschutzglas genannt) auf. Dieses Brillenglas umfasst immer eine Flüssigkristallzelle, resp. LCD, bspw. in Form einer TN-, STN- oder VA-Zelle, mit welcher die optische Transmission des Brillenglases in gewünschter Weise geregelt werden kann. Solche Blendschutzbrillen sind hinlänglich bekannt, bspw. aus der US-5067795, US-2009/0251660 oder US-2009/0213283.

Bei all diesen bekannten Vorrichtungen ist die Elektronik und Stromversorgung im Brillengestell (d.h. in der Brillenfassung oder den Brillenbügeln) untergebracht. Dies führt zu unerwünscht schweren und/oder plumpen Brillengestellformen. Insbesondere wird durch diese Vorrichtungen die Designfreiheit, d.h. Formgebung der Brillenhersteller stark eingeschränkt Darüber hinaus lassen transparente Gestelle das Innenleben erkennen, was nicht immer erwünscht ist. Ausserdem erfordern diese bekannten Blendschutzbrillen eine aufwändige Fertigung (aufwändige Elektronik) und Nachbearbeitung der Brillengestelle, wegen der integrierten elektrischen Leiterbahnen, der erforderlichen Kontaktstellen und der Elektronik, wobei die Batterien zu einer unerwünschten Gewichtserhöhung führen. Darüber hinaus führen Feuchtigkeit und Verschmutzung immer auch zu Korrosionserscheinungen, Fehlfunktionen, etc. Bisherige aktive Optikelemente erweisen sich wegen der erforderlichen Verdrahtung als untauglich für die industrielle Fertigung, d.h. sind nicht massentauglich, weil deren Fertigung immer auch kostenintensiv ist.

Es ist deshalb Aufgabe der vorliegenden Erfindung eine elektrooptische Schutz-, Sport- oder Sonnenbrille zu schaffen, welche die Nachteile der bekannten Blendschutzbrillen nicht aufweist und insbesondere die Gestaltungsfreiheit für das Brillengestell (Brillenbügel, Brillenfassung, etc.) nicht einschränkt und industriell herstellbar ist, gewichtsarm und wasserdicht ist.

Erfindungsgemäss wird diese Aufgabe durch ein elektro-optisches Blendschutzglas für Schutz-, Sport- und Sonnenbrillen mit den Merkmalen des Anspruchs 1 gelöst und insbesondere durch ein Blendschutzglas mit einer elektronischen Regelschaltung für eine LCD mit mindestens einem aussenseitig (Bildseite) und einem innenseitig (Beobachtungsseite) angeordneten Polarisator, wobei die Regelschaltung, mit oder ohne Stromversorgung in der LCD des Blendschutzglases integriert ist, d.h. in einer Kavität im Innern dieses Blendschutzglases angeordnet ist.

Erfindungsgemäss ist das elektro-optische Blendschutzglas für Schutz-, Sport- und Sonnenbrillen geeignet und weist eine miniaturisierte Regelschaltung für eine LCD auf, mit mindestens einem aussenseitig und einem innenseitig des Blendschutzglases angeordneten Polarisator auf, wobei die Regelschaltung in der LCD des Blendschutzglases integriert ist, d.h. in einer Kavität im Innern dieses Blendschutzglases angeordnet ist. Grundsätzlich ist diese Regelschaltung zwischen diesen beiden Polarisatoren angeordnet, wobei bevorzugt die Fotodioden der Regelschaltung direkt hinter dem aussenseitigen Polarisator liegen. In einer bevorzugten Ausführungsform mindestens teilweise in einer transparenten Füllmasse eingegossen ist, insbesondere derart, dass lediglich die Fotosensoren in einer transparenten Füllmasse liegen, während der übrige Teil der Regelschaltung vorzugsweise in einer opaken Füllmasse eingebettet ist.

In einer besonderen Ausführungsform weist die Regelschaltung einen als Relaxations-Oszillator konfigurierten Komparator mit nachgeschaltetem Inverter (little logic inverter gate) und für die lichtabhängige Speisung der Regelschaltung mehrere in Serie geschaltete Fotodioden auf. Die Regelschaltung wird damit batterielos betrieben, d.h. die Sensoren liefern eine variable Stromversorgung. Die Kapazität eines ersten, zu den in Serie geschalteten Fotodioden parallel geschalteten Kondensator ist derart gewählt ist, dass die Transmission des Blendschutzglases entweder eher im hellen Bereich (für Autofahrer) oder eher im Dunkelbereich (für Gletschergänger) liegt.

Es versteht sich, dass bei dem erfindungsgemäss autarken Blendschutzglas die LCD entweder plan ist und zwischen zwei planen Glasplatten angeordnet ist oder gewölbt ist, insbesondere sphärisch gewölbt ist und zwischen zwei gewölbten, insbesondere sphärisch gewölbten Glasplatten angeordnet ist. Die verwendete Optik kann auch mit bifokaler oder anderer optischer Korrekturen versehen sein.

Die Vorteile des erfindungsgemässen Blendschutzglases sind dem Fachmann unmittelbar ersichtlich und sind in der Schaffung eines autarken, elektro-optischen Blendschutzglases zu sehen. Insbesondere lässt sich das Einfügen das elektronischen Teils (Regelschaltung, etc.) in einfacher Weise, d.h. mit geringen Zusatzkosten in den LCD-Herstellungsprozess einfügen. Dadurch können Montage- und Nachbearbeitungskosten (bspw. nachträgliches Verglessen, Polieren, etc.) bei der Herstellung des Brillengestells eingespart werden. Insbesondere kann damit auch eine erhöhte Robustheit und Resistenz gegen Feuchtigkeit, Schmutz und/oder Wasser. Der batterielose Betrieb führt zu einer weiteren Gewichtsreduktion, und damit zu einem gewichtsarmen, elektrooptischen Blendschutzglas.

Der hier verwendete Begriff "Blendschutzglas" soll jede Form von elektro-optischen Brillengläsern umfassen, insbesondere nicht nur solche mit mineralischen Gläsern sondern auch solche mit geeigneten Kunststoffgläsern (bspw. aus Polykarbonat). Diese mineralischen Gläser, resp. Kunststoffgläser können geschliffen, d.h. mit ophthalmischer Wirkung oder einfach plan sein. Im Folgenden sollen diese Gläser auch Substrat, Optik oder Glasplatte genannt werden.
LCDs sind hinlänglich bekannt und umfassen mindestens eine Flüssigkristallschicht, meistens dünner als 10 µm, je mindestens aussen- und innenseitig eine gebürstete Polyimidschicht, je mindestens eine Elektrodenschicht (ITO), je mindestens ein Substrat (bspw. eine dünne Glasplatte) und je mindestens einen Polarisator.
Es versteht sich, dass hier unter dem Begriff Blendschutz jede Art von Schutz vor optischer Blendung zu verstehen ist.

Im Folgenden soll die Erfindung anhand eines Ausführungsbeispiels und mit Hilfe der Figuren näher erläutert werden. Dabei zeigen:
- Fig. 1: räumliche Ansicht einer elektrooptischen Blendschutzbrille bekannter Art;
- Fig. 2: Schaltbild für eine bevorzugten Steuerschaltung für ein erfindungsgemässes Blendschutzglas;
- Fig. 3:: schematische Darstellung eines Querschnitts durch ein erfindungsgemässes Blendschutzglas.

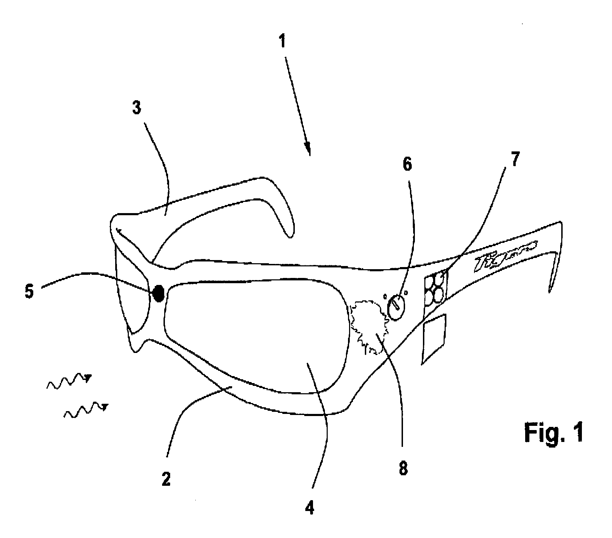

Fig. 1 zeigt eine Blendschutzbrille (1) bekannter Art mit einer relativ wuchtig wirkenden Brillenfassung (2) für die beiden Brillengläser (4). In einem Brillenbügel (3) ist eine Elektronik (8) und ein Set erforderlicher Batterien (7) eingepasst. Ein Sensor (5) für die Steuerung der Elektronik (8) ist im Nasensteg angeordnet. Ein Regler (6) erlaubt die Anpassung der Transmissionseigenschaften der Brillengläser (4).

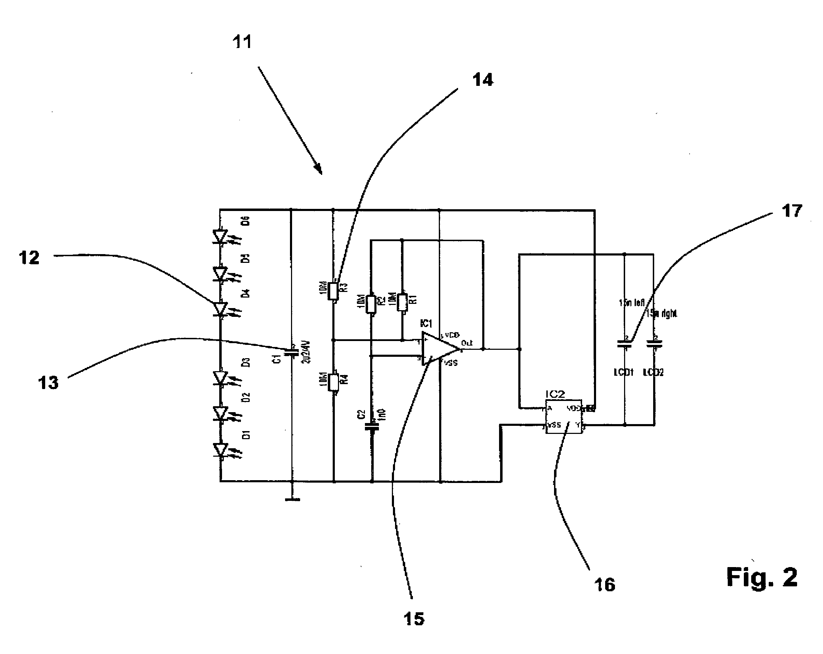

Das in Fig. 2 dargestellte Schaltbild zeigt eine bevorzugte Mini-Regelschaltung (im Folgenden auch nur Elektronik genannt), welche lediglich sechs Fotodioden, einen Komparator, einen Little Logic Inverter, vier Widerstände und zwei Kondensatoren aufweist. Alle Bauteile weisen eine sehr geringe Bauhöhe (höchstens 0.7 mm) auf. In Serie geschaltete Fotodioden dienen als Solarzellen, d.h. als lichtabhängige Stromversorgung. Die Betriebsfrequenz der jeweiligen TN- Flüssigkristallzellen ist so gewählt, dass die gewünschte Regelkurve für eine Blendschutzbrille entsteht. Ein Relaxations-Oszillator mit Push-Pull Ausgang und ein Inverter Gate treiben direkt die LCD. Diese Schaltung startet bei tiefer Beleuchtungsstärke (ca. E = 10'000 lux, entspricht der Beleuchtung an einem grauen Wintertag), d.h. ist so ausgelegt, dass der Oszillator bereits unter der Schwellwertspannung der LCD startet. Damit ist kein Starten der Regelung erkennbar, resp. verdunkelt die LCD kontinuierlich. Diese Mini-Regelschaltung ist kleiner als 4 x 4 x 0.8 mm³, wobei die Fotodioden lediglich ca. 6 mm² benötigen. Es versteht sich, dass diese Fotodioden frei gegen das zu beobachtende Objekt gerichtet sein müssen, während die Elektronik auch hinter bspw. einer Brillenfassung oder einem Designelement (Hindu Punkt, Schweizerkreuz oder Farbstreifen) verborgen sein kann. Die von den Fotodioden erzeugte Energie reicht aus, auch wenn diese unter der einen oder anderen Brillenglasschicht liegen, bspw. unter dem Polarisator.

In einer ersten bevorzugten Ausführungsform der bevorzugten Elektronik treibt ein schon bei 0.6 V funktionsfähiger Relaxations-Oszillator (mit einem Komparator und einem nachgeschaltetem Little Logic Inverter Gate) dank einem Push-Pull-Ausgangssignal direkt die kapazitive Last der beiden LCDs flimmerfrei bei ca. 70 HZ. Der Treiber startet schon unter der Schwellwertspannung der bekannten TN-LCD, welche in diesem Ausführungsbeispiel bei ca. 1.1 V liegt. Dies ermöglicht das sanfte Einsetzen der Abdunkelung, d.h. es ist kein "Schaltpunkt" zu erkennen. Die in Serie geschalteten Fotodioden dienen als variable Speisung. Bei zunehmendem Lichteinfall - ab etwa 1000 lux - beginnt die stetig zunehmende Abdunkelung. Die Schaltung ist so dimensioniert, dass ein leichter Leistungsüberschuss vorliegt, der jedoch durch das Festlegen einer Betriebsfrequenz "vernichtet" wird. Die Regelfunktion ist so festgelegt.

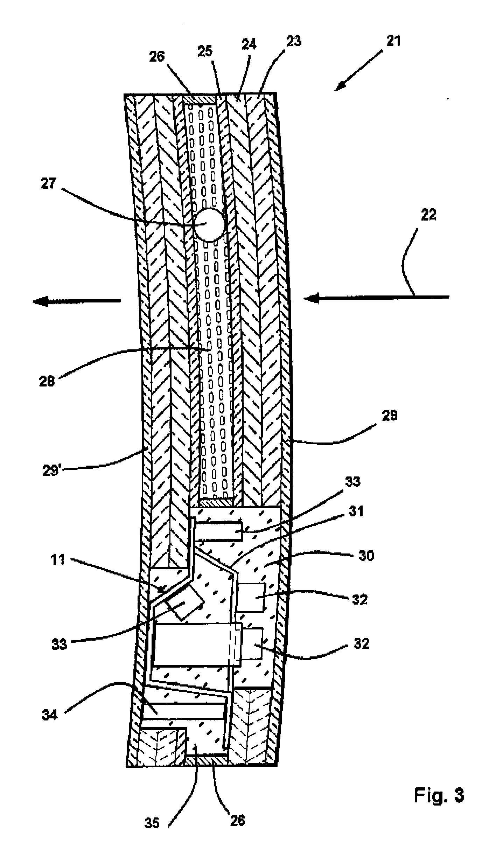

Der in Fig. 3 dargestellte schematische Aufbau eines erfindungsgemäss autarken Blendschutzglases macht deutlich, wie die Kavität (30) mit der Elektronik im Innern des Blendschutzglases angeordnet sein kann. Der grundsätzliche Aufbau einer LCD ist hinlänglich bekannt. Mit der vorliegenden Erfindung kann die jeweilige Flüssigkristallzelle zwischen zwei Planlinsen angeordnet sein oder kann eine gewölbte Flüssigkristallzelle zwischen zwei gewölbten, Glasträgern liegen. Vorzugsweise sind diese Glasträger (Glasplatten, ophthalmische Korrekturgläser) aus einem sehr dünnen Material mit einem hohen Brechungsindex (Polykarbonat n = 1.6 oder Glas n = 1.7) gefertigt. In einer ersten einfachen Ausführungsform werden sphärisch gewölbte Rohlinge mit einem Durchmesser von 60 mm und einem Krümmungsradius von 83 mm, besser 167 mm oder 250 mm verwendet. Für die Herstellung der erfindungsgemässen Blendschutzgläser kommen bekannte Verfahren aus der herkömmlichen LCD-Fertigung zur Anwendung, wie bspw. in DE-19832812 oder EP-1507163 beschrieben. Dabei wird die erfindungsgemässe Kavität für die Elektronik vor dem Einfüllen des Flüssigkristalls eingearbeitet. Diese Verfahrensschritte sind hinlänglich bekannt, d.h. liegen im Bereich des normalen fachmännischen Handelns und sind nicht Gegenstand der vorliegenden Erfindung. Natürlich sind auch andere bekannte Fertigungsverfahren nicht auszuschliessen, bspw. das nachträgliche Krümmen der fertig zusammengesetzten LCD, wie bspw. aus der EP-1428063 bekannt oder die Verwendung von Kunststoffgläsern aus polarisierendem Material, wie bspw. in der DE-3711417 beschrieben.Es versteht sich, dass die Polarisatoren mit zusätzlichen Beschichtungen versehen sein können.

Die erfindungsgemässen Blendschutzgläser sind autark und können deshalb als sogenantes "cover lens glass" verwendet werden, resp. eignen sich besonders für die Verwendung in durchgesetzten Designergestellen.

Es versteht sich, dass das erfindungsgemässe LCD-Blendschutzglas auch in einer planen Form zwischen zwei Planlinsen angeordnet sein kann oder in einer gewölbten Form zwischen zwei gewölbten Linsenoptiken angeordnet sein kann. Ebenso lassen sich gefärbte Gläser und/oder selektiv absorbierende Beschichtungen und/oder AntiReflex- Beschichtung mit dem erfindungsgemässen Blendschutzglas kombinieren und/oder lassen sich die Polarisatoren im Trägerglas integrieren, d.h. lassen sich Substrate und/oder Gläser mit polarisierenden Eigenschaften verwenden und/oder können besondere Flüssigkristalle, bspw. elektrochromische Flüssigkristalle verwendet werden.

Darüber hinaus ist das vorliegende Blendschutzglas auch in anderen optischen Vorrichtungen einsetzbar, insbesondere für die Fotografie, Mikroskopie, optische Erkennungs- und/oder Messsysteme, etc.

### Bezugszeichen:

- 1: Blendschutzbrille
- 2: Brillenfassung
- 3: Brillenbügel
- 4: Brillenglas
- 5: Sensor, Fotodiode
- 6: Regler
- 7: Batterien
- 8: Elektronik
- 11: Regelschaltung
- 12: Fotodioden
- 13: Kondensatoren
- 14: Widerstände
- 15: Komparator
- 16: little logic Gate
- 17: LCDs
- 21: Blendschutzglas
- 22: einfallendes Licht
- 23: Glasplatte
- 24: elektrisch leitende Schicht (ITO, indium-tin-oxyde)
- 25: Orientierungsschicht (alignment layer, Polyimid)
- 26: Verschlusselement (plug, epoxy)
- 27: Anstandshalter (spacer)
- 28: Flüssigkristall
- 29: aussenseitiger Polarisator
- 29': innenseitiger Polarisator
- 30: Kavität
- 31: Leiterplatte
- 32: Fotodiode
- 33: Elektronikbauteil
- 34: Montagehilfe
- 35: Füllmasse

## Patentansprüche

1. Elektro-optisches Blendschutzglas (21), insbesondere für Schutz-, Sport- und Sonnenbrillen, mit einer elektronischen Regelschaltung (11) für eine LCD (17) mit mindestens einem aussenseitig (29) und einem innenseitig angeordneten Polarisator (29'), **dadurch gekennzeichnet, dass** die Regelschaltung (11) in der LCD (17) des Blendschutzglases (21) integriert ist, d.h. in einer Kavität (30) im Innern dieses Blendschutzglases (21) angeordnet ist.

2. Elektro-optisches Blendschutzglas nach Anspruch 1, **dadurch gekennzeichnet, dass** die Regelschaltung (11) mehrere Fotodioden (12) aufweist und zwischen diesen beiden Polarisatoren (29,29') der LCD (17) angeordnet ist.

3. Elektro-optisches Blendschutzglas nach Anspruch 2, **dadurch gekennzeichnet, dass** die Fotodioden (32) der Regelschaltung (11) direkt hinter dem aussenseitigen Polarisator (29) liegen.

4. Elektro-optisches Blendschutzglas nach Anspruch 1, **dadurch gekennzeichnet, dass** die Regelschaltung (11) mindestens teilweise in einer transparenten Füllmasse (35) eingegossen ist.

5. Elektro-optisches Blendschutzglas nach einem der Ansprüche 1 bis 4, **dadurch gekennzeichnet, dass** die Regelschaltung (11) einen als Relaxations-Oszillator konfigurierten Komparator (15) mit nachgeschaltetem Inverter (16, little logic inverter gate) aufweist.

6. Elektro-optisches Blendschutzglas nach Anspruch 1, **dadurch gekennzeichnet, dass** dieses batteriefrei ist, d.h. für die Speisung der Regelschaltung (11) die mehreren, vorzugsweise sechs in Serie geschalteten, Fotodioden (12) vorgesehen sind.

7. Elektro-optisches Blendschutzglas nach Anspruch 6, **dadurch gekennzeichnet, dass** ein erster zu den in Serie geschalteten Fotodioden (12) parallel geschalteter Kondensator (13) derart gewählt ist, dass die Transmission des Blendschutzgases (21) entweder eher im hellen Bereich (für Autofahrer) oder eher im Dunkelbereich (für Gletschergänger) liegt.

8. Elektro-optisches Blendschutzglas nach Anspruch 1, **dadurch gekennzeichnet, dass** die LCD (17) plan ist und zwischen zwei planen Glasplatten (23) angeordnet ist.

9. Elektro-optisches Blendschutzglas nach Anspruch 1, **dadurch gekennzeichnet, dass** die LCD (17) gewölbt, insbesondere sphärisch gewölbt ist und zwischen zwei gewölbten, insbesondere sphärisch gewölbten Glasplatten (23) angeordnet ist.

10. Elektro-optisches Blendschutzglas nach Anspruch 2, **dadurch gekennzeichnet, dass** dieses in einer Schutz-, Sport- oder Sonnenbrille oder in einer anderen optischen Vorrichtung eingesetzt ist.
